(19) Europäisches Patentamt
European Patent Office
Office européen des brevets

(11) Publication number: **0 047 057**
**B1**

# (12) EUROPEAN PATENT SPECIFICATION

(45) Date of publication of patent specification: **18.09.85**

(51) Int. Cl.[4]: **C 07 D 249/08,** A 01 N 43/64, C 07 C 29/36, C 07 D 303/28

(21) Application number: **81303272.9**

(22) Date of filing: **16.07.81**

## (54) Triazole compounds, a process for preparing them, their use as plant fungicides and fungicidal compositions containing them.

(30) Priority: **26.08.80 GB 8027558**
**08.12.80 GB 8039277**

(43) Date of publication of application:
**10.03.82 Bulletin 82/10**

(45) Publication of the grant of the patent:
**18.09.85 Bulletin 85/38**

(84) Designated Contracting States:
**AT BE CH DE FR GB IT LI LU NL SE**

(56) References cited:
**EP-A-0 004 315**
**EP-A-0 010 674**
**EP-A-0 015 756**
**FR-A-1 408 958**
**GB-A-1 448 437**
**GB-A-1 529 818**
**US-A-3 800 044**

**The file contains technical information submitted after the application was filed and not included in this specification**

(73) Proprietor: **IMPERIAL CHEMICAL INDUSTRIES PLC**
**Imperial Chemical House Millbank**
**London SW1P 3JF (GB)**

(72) Inventor: **Worthington, Paul Anthony**
**4 Oakhurst Road Maidenhead Court Park**
**Maidenhead Berkshire (GB)**
Inventor: **De Fraine, Paul**
**77 McCarthy Way**
**Wokingham Berkshire (GB)**
Inventor: **Rathmell, William George**
**'Culvers' 10 Gypsy Lane**
**Wokingham Berkshire (GB)**

(74) Representative: **Alner, Henry Giveen Hamilton et al**
**Imperial Chemical Industries PLC Legal Department: Patents P.O. Box 6 6, Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 047 057 B1

## Description

This invention relates to triazole compounds useful as fungicides, to a process for preparing them, to fungicidal compositions containing them, and to processes using them to combat fungal infections in plants. The invention also relates to pharmaceutical and veterinary compositions comprising triazole compounds, and in particular to such compositions which are orally or topically active against fungus diseases of humans and other animals. These compositions of the invention are especially useful for treatment of candidiasis and human dermatophyte infections.

The invention provides a compound having the formula:

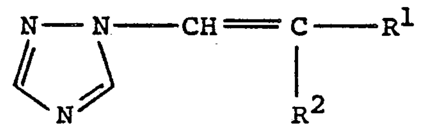

formula (I)

which is the cis- or trans- isomer, or a mixture thereof, wherein $R^1$ and $R^2$, which may be the same or different, are alkyl having from 1 to 6 carbon atoms, cycloalkyl having up to 6 carbon atoms (eg. cyclopropyl, cyclopentyl, or cyclohexyl) or phenyl optionally substituted with halogen, alkyl or halo-alkyl having from 1 to 5 carbon atoms, alkoxy or halo-alkoxy having from 1 to 4 carbon atoms, nitro, phenyl or phenoxy; an acid addition salts and metal complexes thereof.

The compounds of the invention are generally obtained in the form of isomeric mixtures which can be separated into the individual isomers by methods known in the art.

The alkyl groups can be a straight or branched chain group having 1 to 6, e.g. 1 to 4, carbon atoms; examples are methyl, ethyl, propyl (*n*- or *iso*-propyl) and butyl *n-sec-, iso-* or *t*-butyl).

Examples of suitable substituents for the phenyl are halogen (e.g. fluorine, chlorine or bromine), $C_{1-5}$ alkyl [e.g. methyl, ethyl, propyl (*n*- or *iso*-propyl) and butyl (*n*-, *sec*-, *iso*-or *t*-butyl)], $C_{1-4}$ alkoxy (e.g. methoxy and ethoxy), $C_{1-4}$ halo-alkoxy (e.g. trifluoromethoxy), $C_{1-5}$ halo-alkyl (e.g. trifluoromethyl), nitro, phenyl and phenoxy.

Either $R^1$ or $R^2$, but preferably both $R^1$ and $R^2$, are phenyl, unsubstituted or substituted with 1, 2 or 3 ring substituents as defined above. Preferably the phenyl has one or two ring substituents in the 2- or 4-positions. Examples of these groups are phenyl, 2-, 3- or 4-chlorophenyl, 2,4- or 2,6-dichlorophenyl, 2-, 3- or 4- fluorophenyl, 2,6-difluorophenyl, 2-, 3- or 4-bromophenyl, 2-chloro-4-fluorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-, 3- or 4-methylphenyl, 2-, 3- or 4-*t*-butylphenyl, 2-, 3- or 4-trifluoromethylphenyl, 2-, 3- or 4-phenoxyphenyl, and 2-, 3- or 4-phenylphenyl (2-, 3- or 4-biphenylyl).

In a further aspect, therefore, the invention provides a triazole compound as defined above wherein $R^1$ and $R^2$, which may be same or different are straight or branched chain alkyl groups having from 1 to 6 carbon atoms, or are phenyl optionally substituted with halogen, alkyl or haloalkyl having from 1 to 5 carbon atoms, alkoxy or halo-alkoxy having from 1 to 4 carbon atoms, nitro, phenyl or phenoxy.

In a still further aspect the invention provides a triazole compound as defined above wherein $R^1$ and $R^2$ are alkyl having from 1 to 4 cabon atoms, phenyl or halophenyl.

In a preferred aspect the invention provides a triazole compound as defined above wherein $R^1$ and $R^2$ are both phenyl, 2-, 3- or 4-chlorophenyl, 2, 4- or 2,6-dichlorophenyl, 2-, 3- or 4-fluorophenyl, 2,6-difluorophenyl, 2-, 3- or 4-bromophenyl, 2-chloro-4-fluorophenyl, 2-chloro-6-fluorophenyl, 2-, 3- or 4-methoxyphenyl, 2,4-dimethoxyphenyl, 2-, 3- or 4-ethoxyphenyl, 2-, 3- or 4-nitrophenyl, 2-, 3- or 4-trifluoromethylphenyl, 2-, 3- or 4-phenoxyphenyl, or 2-, 3- or 4-phenylphenyl (2-, 3- or 4-biphenyl).

The salts can be salts with inorganic or organic acids e.g. hydrochloric, nitric, sulphuric, acetic, 4-toluene-sulphonic or oxalic acid.

Suitably the metal complex is one including, as the metal, copper, zinc, manganese or iron. It preferably has the general formula:

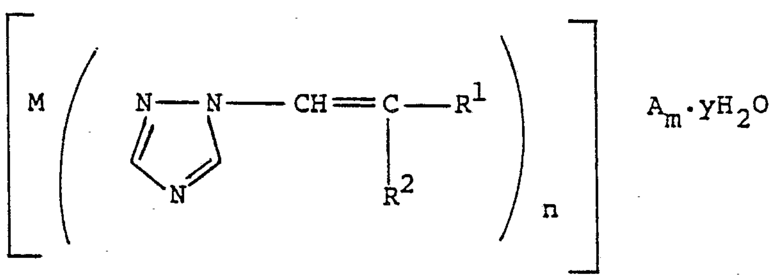

wherein $R^1$ and $R^2$ are as defined above, M is a metal, A is an anion (e.g. a chloride, bromide, iodide, nitrate sulphate or phosphate anion), n is 2 or 4, y is 0 or an integer of 1 to 12, and m is an integer consistent with valency.

Examples of the compounds of the invention are shown in Table I.

TABLE I

| Compound No | $R^1$ | $R^2$ | Melting Point (°C) |
|---|---|---|---|
| 1 | 2-chlorophenyl | 4-fluorophenyl | Oil |
| 2 | 2-chlorophenyl | 4-fluorophenyl | (nitrate salt of 1) 124 |
| 3 | phenyl | phenyl | |
| 4 | 4-chlorophenyl | 4-chlorophenyl | |
| 5 | 2,4-dichlorophenyl | phenyl | |
| 6 | 2-fluorophenyl | 4-chlorophenyl | |
| 7 | 2-fluorophenyl | 4-fluorophenyl | |
| 8 | 2-chlorophenyl | 4-chlorophenyl | |
| 9 | *t*-butyl | 4-chlorophenyl | |
| 10 | *t*-butyl | *t*-butyl | |
| 11 | 2-bromophenyl | 4-chlorophenyl | nitrate salt 140—1 |
| 12 | 2-bromophenyl | 4-fluorophenyl | nitrate salt 124—5 |
| 13 | 4-chlorophenyl | 4-fluorophenyl | 101—2 |
| 14 | 4-fluorophenyl | 4-fluorophenyl | |
| 15 | 2-chloro-4-methoxyphenyl | 4-fluorophenyl | |
| 16 | 4-(4-chlorophenyl)phenyl | phenyl | |
| 17 | 4-(4-chlorophenyl)phenyl | 4-chlorophenyl | |
| 18 | 2,4-dichlorophenyl | 4-fluorophenyl | |
| 19 | 2-chloro-4-methylphenyl | 4-fluorophenyl | |
| 20 | *t*-butyl | 2,4-dichlorophenyl | |
| 21 | 2-(5-chlorothienyl) | 2,4-dichlorophenyl | |

The compounds of general formula (I) may be produced by dehydrating a compound of general formula (II):

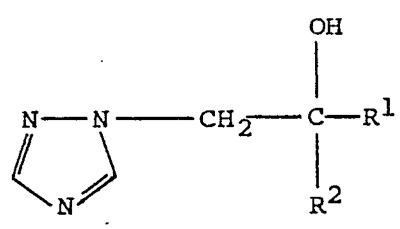

formula (II)

in which $R^1$ and $R^2$ are as defined above, under acidic conditions by refluxing in a solvent. Suitably the compound of general formula (II) is reacted with phosphoric acid or trifluoroacetic acid by reflux in toluene or other suitable solvent. The product can be isolated by pouring the reaction mixture into water and purification by column chromatography. The compounds are usually produced as oils which readily form salts, e.g. nitrate salts.

The compounds of general formula (II) may be produced by reacting a compound of general formula (III) or (IV):

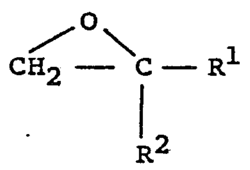

formula (III)

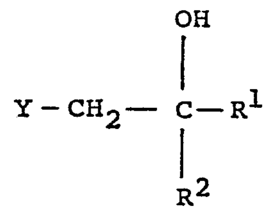

formula (IV)

in which $R^1$ and $R^2$ are as defined above and Y is a halogen atom (preferably a chlorine or bromine atom), with 1,2,4-triazole either in the presence of an acid-binding agent or in the form of one of its alkali metal salts in a solvent.

Suitably the compound of general formula (III) or (IV) is reacted at 20—100°C with the sodium salt of 1,2,4-triazole (the salt can be prepared by adding either sodium hydride or sodium methoxide to 1,2,4-triazole) in a solvent such as acetonitrile, methanol, ethanol or dimethylformamide. The product can be isolated by pouring the reaction mixture into water and recrystallising the solid formed from a solvent.

The compounds of general formula (III) and (IV) can be prepared by reacting a ketone of general formula (Va) or (Vb):

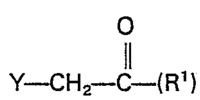

(Va)

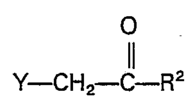

(Vb)

wherein $R^1$, $R^2$ and Y are as defined above with, respectively, a Grignard compound of general formula (VIa) or (VIb):

$Z—Mg—R^2$

(VIa)

$Z—Mg—R^1$

(VIb)

wherein $R^1$ and $R^2$ are as defined above and Z is a halogen (preferably chlorine, bromine or iodine) in a solvent such as diethyl ether or tetrahydro-furan. Generally a mixture of the compounds of general formula (III) and (IV) are obtained. For example, when a compound of general formula (Va) wherein $R^1$ is alkyl or cycloalkyl is reacted, the compound of formula (III) generally predominates in the mixture; on the other hand, when $R^1$ is optionally substituted phenyl, the compound of general formula (IV) generally predominates in the mixture.

The compounds (III) and (IV) are valuable intermediates and form part of the present invention.

The compounds of general formula (V) and (VI) may be made by methods set out in the literature.

The compounds of general formula (III) wherein each of $R^1$ and $R^2$, which may be the same or different, is substituted phenyl may also be prepared by reacting the appropriate ketone of general formula (VII)

$$R^1—CO—R^2 \qquad (VII)$$

wherein $R^1$ and $R^2$ are as defined above, with dimethyl oxosulphonium methylide (Corey and Chaykovsky, JACS, 1965, *87*, 1353—1364) or dimethyl sulphonium methylide (Corey and Chaykovsky, JACS, 1962, *84*, 3782) using methods set out in the literature.

The ketones of general formula (VII) can be prepared, using the Friedel-Crafts reaction, by methods set out in the literature. These ketones are valuable intermediates and as such form part of the invention.

The compounds of general formula (III) wherein each of $R^1$ is alkyl, cycloalkyl or optionally substituted phenyl and $R^2$ is optionally substituted phenyl or optionally substituted benzyl can also be produced by reacting a β-hydroxy selenide compound of the general formula (VIII)

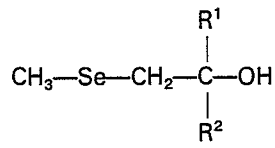

(VIII)

wherein $R^1$ and $R^2$ are as defined above, with methyl iodide in potassium *t*-butoxide according to the method of Van Ende, Dumont and Krief, Angew. Chem, Int. Ed., 1975, *14*, 700.

The β-hydroxy selenide compound can be prepared by treating the diselenide with the appropriate ketone in the presence of butyl lithium.

The salts and metal complexes of the compounds of general formula (I) can be prepared from the latter in known manner. For example, the complexes can be made by reacting the uncomplexed compound with a metal salt in a solvent.

The compounds, salts and metal complexes are active fungicides, particularly against the diseases:—

*Piricularia oryzae* on rice

*Puccinia recondita, Puccinia striiformis* and other rusts on wheat, *Puccinia hordei, Puccinia striiformis* and other rusts on barley, and rusts on other hosts e.g. coffee, apples, vegetables and ornamental plants

*Plasmopara viticola* on vines

*Erysiphe graminis* (powdery mildew) on barley and wheat and other powdery mildews on various hosts such as

*Sphaerotheca fuliginea* on cucurbits (e.g. cucumber),

*Podosphaera leucotricha* on apples and *Uncinula necator* on vines

*Helminthosporium* spp. and *Rhynchosporium* spp. on cereals

*Cercospora arachidicola* on peanuts and other *Cercospora* species on for example sugar beet, bananas and soya beans

*Botrytis cinerea* (grey mould) on tomatoes, strawberries, vines and other hosts

*Phytophthora infestans* (late blight) on tomatoes

*Venturia inaequalis* (scab) on apples

Some of the compounds have also shown a broad range of activities against fungi *in vitro*. They have activity against various post-harvest diseases on fruit (e.g. *Penicillium digatatum* and *italicum* on oranges and *Gloeosporium musarum* on bananas). Further some of the compounds are active as seed dressings against: *Fusarium* spp., *Septoria* spp., *Tilletia* spp. (i.e. bunt, a seed borne disease of wheat), *Ustilago* spp.,*Helminthosporium* spp. on cereals, *Rhizoctonia solani* on cotton and *Corticium sasakii* on rice.

The compounds can move acropetally in the plant tissue. Moreover, the compounds can be volatile enough to be active in the vapour phase against fungi on the plant.

The compounds are also useful for the treatment of candidiasis and human dermatophyte infections.

The compounds may be used as such for fungicidal purposes but are more conveniently formulated into compositions for such usage. The invention thus provides also a fungicidal composition comprising a compound of general formula (I) or a salt or complex thereof as hereinbefore defined, and a carrier or diluent.

The invention also provides a method of combating fungal diseases in a plant, which method comprises applying to the plant, to seed of the plant or to the locus of the plant or seed a compound or a salt or complex thereof as hereinbefore defined.

The compounds, salts and complexes can be applied in a number of ways, for example they can be formulated or unformulated, directly to the foliage of a plant, to seeds or to other medium in which plants are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a

wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a microencapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quarternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene-sulphonate, and a mixture of sodium diisopropyl- and triisopropyl-naphthalene sulphonates.

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for exmaple gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogeneous for a sufficent time to enable them to be applied by conventional spray equipment. The concentrates may conveniently contain up to 95%, suitably 10—85%, for example 25—60%, by weight of the active ingredient(s). These concentrates suitably contain organic acids (e.g. alkaryl or aryl sulphonic acids such as xylenesulphonic acid or dodecyl benzenesulphonic acid) since the presence of such acids can increase the solubility of the active ingredient(s) in the polar solvents often used in the concentrates.

The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal activity or compounds having plant growth regulating, herbicidal or insecticidal activity.

The other fungicidal compound can be for example one which is capable of combating ear diseases of cereals (e.g. wheat) such as *Septoria, Gibberella* and *Helminthosporium* spp., seed and soil borne diseases and downy and powdery mildews on grapes and powdery mildew and scap on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazitine, dodine, fentin acetate, fentin hydroxide, dinicap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenapanil, ofurace, propiconazole, etaconazole and fenpropemorph.

The compounds of general formula (I) can be mixed with soil, peat or other rooting media for the protection of plants against seed-borne, soil-borne or foliar fungal diseases.

Suitable insecticides are pirimicarb, ethiofencarb, dimethoate, dimeton-s-methyl and formothion.

Examples of suitable plant growth regulating compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyl-adenine or benzylaminopurine), phenoxyacetic acids (eg. 2,4-D or MCPA), substituted benzoic acids (eg. triiodobenzoic acid), morphactins (eg. chlorofluorecol) maleic hydrazide, glyphosphate, glyphosine, long chain fatty alcohols and acids, dikegulac, fluoridamid, mefluidide, substituted quarternary ammonium and phosphonium compounds (eg. chlormequat or chlorphonium), ethepon, carbetamide, methyl-3,6-dichloranisate, diaminozide, asulam, abscissic acid, isopyrimol, 1(4-chlorophenyl)-4,6-dimethyl-2-oxo-1,2-dihydropyridine-3-carboxylic acid, hydroxybenzo-nitriles (eg. bromoxynil), difenzoquat, benozylprop-ethyl 3,6-dichloropicolinic acid.

As indicated above the compounds of the invention possess antifungal properties which are useful in the treatment of candidiasis and human dermatophyte infections. For this purpose they are conveniently formulated into pharmaceutical and verterinary compositions.

The pharmaceutical and veterinary compositions of the invention may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions of the invention are compositions suitable for oral administration, and particularly tablets and capsules.

Thus according to a further feature of the invention there is provided a pharmaceutical or veterinary fungicidal composition which comprises a compound of formula I as defined above, or a salt, metal complex, ether or ester thereof, together with a pharmaceutically or veterinary acceptable diluent or carrier.

The following Examples illustrate the invention; the temperatures are given in degrees Centigrade (°C).

## Example 1

This Example illustrates the preparation of:
1-(1,2,4-Triazol-1-yl)-2-(2-chlorophenyl)-2-(4-fluorophenyl)ethylene (Compound No 1 of Table I) and its nitrate salt (Compound No 2 of Table I)

A solution of dimethyl oxosulphonium methylide was prepared under nitrogen from sodium hydride (0.03 mol) and powdered trimethyl oxosulphonium iodide (0.03 mol) in dry dimethylsulphoxide (DMSO; 30 ml). A solution of 2-chlorophenyl-4-fluorophenyl ketone (0.025 mol) in DMSO (10 ml) was added dropwise at room temperature. The solution was then heated at 50° for 1.5 hours, cooled to room temperature and poured into water. The solution was extracted with diethyl ether (100 ml), washed with water (3 × 100 ml), and dried over anhydrous sodium sulphate. Removal of the solvent gave 1-(2-chlorophenyl)-1-(4-fluorophenyl)ethylene oxide (90%) as a colourless liquid.

1,2,4-Triazole (0.04 mol) was added portionwise to sodium hydride (0.04 mol) in dimethylformamide (DMF) (40 ml) and the solution stirred at room temperature until effervescence ceased. 1-(2-Chlorophenyl)-1-(4-fluorophenyl)ethylene oxide (0.02 mol) in DMF (10 ml) was added dropwise and the solution stirred at 80° for 4 hours. The solution was poured into water and triturated with petroleum ether to give a white crystalline solid which was filtered off and dried. Recrystallisation from petroleum ether (60—80°)/methylene chloride gave 1-(1,2,4-Triazol-1-yl)-2-(2-chlorophenyl)-2-(4-fluorophenyl)-ethan-2-ol (70%) as a white crystalline solid m.p. 115—6°.

A mixture of 1-(1,2,4-Triazol-1-yl)-2-(2-chlorophenyl)-2-(4-fluorophenyl)ethan-2-ol (0.01 mol) and orthophosphoric acid (30 ml) were stirred at room temperature for 30 minutes and then warmed at 50° for 1 hour. After standing overnight at room temperature the mixture was poured into water (200 ml) and extracted with diethyl ether (150 ml). The ether solution was washed with water (3 × 100 ml) and dried over anhydrous sodium sulphate.

Removal of the solvent gave a yellow oil which was purified by medium pressure column chromatogrpahy (silica gel eluted with ethyl acetate/petrol 1:1) to give the title compound as a light brown oil (80%). The oil was dissolved in diethyl ether (30 ml) and concentrated $HNO_3$ (6 drops) added to precipitate out the title compounds as the nitrate salt m.p. 124°.

## Example 2

This Example illustrates the preparation of:
1-(1,2,4-Triazol-1-yl)-2-(4-chlorophenyl)-2-(4-fluorophenyl)ethylene (Compound No 13 of Table I)

A solution of dimethyl oxosulphonium methylide was prepared under nitrogen from sodium hydride (0.03 mol) and powdered trimethyl oxosulphonium iodide (0.03 mol) in dry dimethyl sulphoxide (DMSO; 30 ml). A solution of 4-chlorophenyl-4-fluorophenyl ketone (0.025 mol) in DMSO (10 ml) was added dropwise at room temperature. The solution was then heated at 50° for 1.5 hours, cooled to room

temperature and poured into water. The solution was extracted with diethyl ether (100 ml), washed with water (3 × 100 ml), and dried over anhydrous sodium sulphate. Removal of the solvent gave 1-(4-chlorophenyl)-1-(4-fluorophenyl)ethylene oxide (90%) as a colourless oil.

1,2,4-Triazole (0.04 mol) was added portionwise to sodium hydride (0.04 mol) in DMF (40 ml) and the solution stirred at room temperature until effervescence ceased. 1-(4-chlorophenyl)-1-(4-fluorophenyl)-ethylene oxide (0.02 mol) in DMF (10 ml) was added dropwise and the solution stirred at 80° for 4 hours. The solution was poured into water and triturated with petroleum ether to give a white crystalline solid which was filtered off and dried. Recrystallisation from petroleum ether (60—80°)/chloroform gave 1-(1,2,4-Triazol-1-yl)-2-(4-chlorophenyl)-2-(4-fluorophenyl)ethan-2-ol (75%) as a white crystalline solid m.p. 154—5°.

A mixture of 1-(1,2,4-Triazol-1-yl)-2-(4-chlorophenyl)-2-(4-fluorophenyl)ethan-2-ol (0.007 mol) and orthophosphoric acid (30 ml) were stirred at room temperature for 30 minutes and warmed to 50° for 1 hour. After standing overnight at room temperature the mixture was poured into water (200 ml), extracted with ether (150 ml) and washed with saturated sodium bicarbonate solution. The ether solution was washed with water (2 × 100 ml) and dried over anhydrous sodium sulphate. Removal of the solvent gave a solid which recrystallised from petroleum ether (60—80°)/chloroform as the title compound (50%) m.p. 101—2°.

Example 3

An emulsifiable concentrate was made up by mixing the ingredients, and stirring the mixture until all the constituents were dissolved.

| | |
|---|---|
| Compound No 1 of Table I | 10% |
| Ethylene dichloride | 40% |
| Calcium dodecylbenzenesulphate | 5% |
| "Lubrol" L | 10% |
| "Aromasol" H | 35% |

Example 4

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, to obtain the desired size of grains.

| | |
|---|---|
| Compound No 2 of Table I | 50% |
| "Dispersol" T | 25% |
| "Lubrol" APN5 | 1.5% |
| Sodium acetate | 23.5% |

Example 5

The ingredients were all ground together to produce a powder formulation readily dispersible in liquids.

| | |
|---|---|
| Compound No 11 of Table I | 45% |
| "Dispersol" T | 5% |
| "Lissapol" NX | 0.5% |
| "Cellofas" B600 | 2% |
| Sodium acetate | 47.5% |

Example 6

The active ingredient was dissolved in a solvent and the resultant liquid was sprayed on to the granules of China clay. The solvent was then allowed to evaporate to produce a granular composition.

| | |
|---|---|
| Compound No 12 of Table I | 5% |
| China clay granules | 95% |

Example 7

A composition suitable for use as a seed dressing was prepared by mixing the three ingredients.

| | |
|---|---|
| Compound No 13 of Table I | 50% |
| Mineral oil | 2% |
| China clay | 48% |

Example 8

A dusting powder was prepared by mixing the active ingredient with talc.

| | |
|---|---|
| Compound No 1 of Table I | 5% |
| Talc | 95% |

Example 9

A Col formation was prepared by ball-milling the constituents set out below and then forming an aqueous suspension of the ground mixture with water.

| | |
|---|---|
| Compound No 2 of Table I | 40% |
| "Dispersol" T | 10% |
| "Lubrol" APN5 | 1% |
| Water | |

Example 10

A dispersible powder formulation was made by mixing together the ingredients set out below and then grinding the mixture until all were thoroughly mixed.

| | |
|---|---|
| Compound No 11 of Table I | 25% |
| "Aerosol" OT/B | 2% |
| "Dispersol" A.C. | 5% |
| China clay | 28% |
| Silica | 40% |

Example 11

The Example illustrates the preparation of a dispersible powder formulation. The ingredients were mixed and the mixture then ground in a comminution mill.

| | |
|---|---|
| Compound No 12 of Table I | 25% |
| "Perminal" BX | 1% |
| "Dispersol" T | 5% |
| Polyvinylpyrrolidone | 10% |
| Silica | 25% |
| China clay | 34% |

Example 12

The ingredients set out below were formulated into a dispersible powder by mixing then grinding the ingredients.

| | |
|---|---|
| Compound No 13 of Table I | 25% |
| "Aerosol" OT/B | 2% |
| "Dispersol"® A | 5% |
| China clay | 68% |

In Examples 3 to 12 the proportions of the ingredients given are by weight.

There now follows an explanation of the compositions or substances represented by the various Trade Marks mentioned above.

LUBROL L:
a condensate of nonyl phenol (1 mole) with ethylene oxide (13 moles)
AROMASOL H:
a solvent mixture of alkylbenzenes
DISPERSOL T & AC:
a mixture of sodium sulphate and a condensate of formaldehyde with sodium naphthalene sulphonate
LUBROL APN5:
a condensate of nonyl phenol (1 mole) with naphthalene oxide (5.5 moles)
CELLOFAS B600:
a sodium carboxymethyl cellulose thickener
LISSAPOL NX:
a condensate of nonyl phenol (1 mole) with ethylene oxide (8 moles)
AEROSOL OT/B:
dioctyl sodium sulphosuccinate
PERMINAL BX:
a sodium alkyl naphthalene sulphonate

Example 13

The compounds were tested against a variety of foliar fungal diseases of plants. The technique employed was as follows.

The plants were grown in John Innes Potting Compost (No 1 or 2) in 4 cm diameter minipots. A layer of fine sand was placed at the bottom of the pots containing the dicotyledonous plants to facilitate uptake of test compound by the roots. The test compounds were formulated either by bead milling with aqueous Dispersol T or as a solution in acetone or acetone/ethanol which was diluted to the required concentration immediately before use. For the foliage diseases, suspensions (100 ppm active ingredient) were sprayed on to the soil. Exceptions to this were the tests on *Botrytis cinerea, Plasmopara viticola* and *Venturia inaequalis.* The sprays were applied to maximum retention and the root drenches to a final concentration equivalent to approximately 40 ppm a.i./dry soil. Tween® 20, to give a final concentration of 0.05%, was added when the sprays were applied to cereals.

For most of the tests the compound was applied to the soil (roots) and to the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment.

The disease control was recorded by the following grading:—

4 = no disease
3 = trace — 5% of disease on untreated plants
2 = 6—25% of disease on untreated plants
1 = 26—59% of disease on untreated plants
o = 60—100% of disease on untreated plants

The results are shown in Table II.

TABLE II

| COMPOUND NUMBER | PUCCINIA RECONDITA (WHEAT) | ERYSIPHE GRAMINIS (BARLEY) | PIRICULARIA ORYZAE (RICE) | PLASMOPARA VITICOLA (VINE) | PHYTOPHTHORA INFESTANS (TOMATO) | BOTRYTIS CINEREA (TOMATO) | CERCOSPORA ARACHIDICOLA (PEANUT) | VENTURIA INAEQUALIS (APPLE) |
|---|---|---|---|---|---|---|---|---|
| 1 | 4 | 4 | — | 0 | 0 | 3 | 4 | 4 |
| 11 | 2 | 4 | — | 3 | 0 | 0 | 3 | 3 |
| 12 | 4 | 4 | — | 4 | 0 | 2 | 4 | 4 |
| 13 | 2 | 4 | — | 0 | 0 | 0 | 1 | 1 |

"—" means not tested.

**Claims**

1. A triazole compound having the general formula (I):

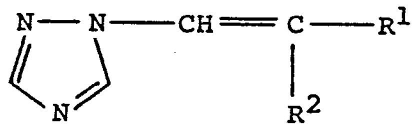

formula (I)

which is the cis- or trans-isomer, or a mixture thereof, wherein $R^1$ and $R^2$, which may be the same or different, are alkyl having from 1 to 6 carbon atoms, cycloalkyl having up to 6 carbon atoms (eg. cyclopropyl, cyclopentyl, or cyclohexyl) or phenyl optionally substituted with halogen, alkyl or halo-alkyl having from 1 to 5 carbon atoms, alkoxy or halo-alkoxy having from 1 to 4 carbon atoms, nitro, phenyl or phenoxy; and acid addition salts and metal complexes thereof.

2. A triazole compound according to claim 1 wherein $R^1$ and $R^2$ are alkyl having from 1 to 4 carbon atoms, phenyl or halo-phenyl.

3. A triazole compound according to claim 1 or 2 wherein $R^1$ and $R^2$ are both phenyl, 2-, 3- or 4-chlorophenyl, 2,4- or 2,6-dichlorophenyl, 2-, 3- or 4-fluorophenyl, 2,6-difluorophenyl, 2-, 3- or 4-bromophenyl, 2-chloro-4-fluorophenyl, 2-chloro-6-fluorophenyl, 2-, 3- or 4-methoxyphenyl, 2,4-dimethoxyphenyl, 2-, 3- or 4-ethoxy-phenyl, 2-, 3- or 4-nitrophenyl, 2-, 3- or 4-trifluoromethyl-phenyl, 2-, 3- or 4-phenoxyphenyl, or 2-, 3- or 4-phenylphenyl (2-, 3- or 4-biphenyl).

4. A process for preparing a compound as defined in any of claims 1 to 3 which comprises dehydrating a compound of general formula (II):

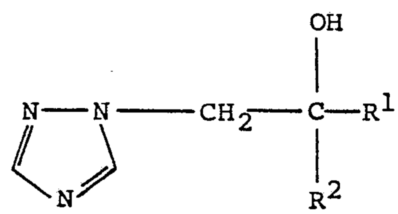

formula (II)

in which $R^1$ and $R^2$ are as defined in any of claims 1 to 3 under acidic conditions by refluxing in a solvent.

5. A process according to claim 5 wherein compounds of general formula (II) are produced by reacting a compound of general formula (III) or (IV):

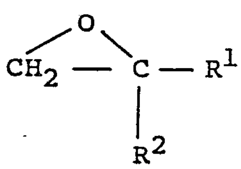

formula (III)

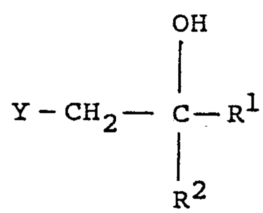

formula (IV)

in which $R^1$ and $R^2$ are as defined in any of claims 1 to 3 and Y is a halogen atom with 1,2,4-triazole either in the presence of an acid-binding agent or in the form of one of its alkali metal salts in a solvent.

6. A process according to claim 5 wherein the compound of general formula (III) or (IV) is reacted at 20—100°C with the sodium salt of 1,2,4-triazole (the salt can be prepared by adding either sodium hydride or sodium methoxide to 1,2,4-triazole) in a solvent such as acetonitrile, methanol, ethanol or dimethylformamide and isolating the product by pouring the reaction mixture into water and recrystallizing.

8. A process according to claim 5 wherein the compounds of general formula (III) and (IV) are prepared by reacting a compound of general formula (Va) or (Vb):

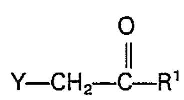

formula (Va)

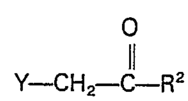

formula (Vb)

wherein $R^1$, $R^2$ and Y are as defined with, respectively, a Grignard compound of general formula (VIa) or (VIb):

$Z—Mg—R^1$

formula (VIa)

$Z—Mg—R^2$

formula (VIb)

wherein $R^1$ and $R^2$ are as defined above and Z is a halogen (preferably chlorine, bromine or iodine) in a solvent such as diethyl ether or tetrahydro-furan.

8. A process according to claim 5 wherein the compounds of general formula (III) wherein each of $R^1$ and $R^2$, which may be the same or different, is substituted phenyl are prepared by reacting the appropriate benzophenone compound of general formula (VII)

$R^1—CO—R^2$ formula (VII)

wherein $R^1$ and $R^2$ are as defined above, with dimethyl oxosulphonium methylide or dimethyl sulphonium methylide.

9. A fungicidal composition comprising as an active ingredient a compound according to any of claims 1 to 3, or a salt or complex thereof; and a carrier or diluent therefor.

10. A method of combating fungal diseases in a plant, which method comprises applying to the plant, to seed of the plant, or to the locus of the plant or seed, a compound, or a salt or complex thereof, as defined in any of claims 1 to 3 or a composition according to claim 9.

11. A pharmaceutical or veterinary fungicidal composition which comprises a compound, or a salt, metal complex, ether or ester thereof, as defined in any of claims 1 to 3 together with a pharmaceutically or veterinary acceptable diluent or carrier.

**Patentansprüche**

1. Triazolverbindung der allgemeinen Formel (I)

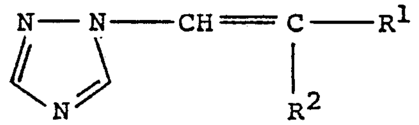

formel (I)

die das cis- oder trans-Isomer oder ein Gemisch davon ist, worin $R^1$ und $R^2$, die gleich oder verschieden sein können, für Alkyl mit 1 bis 6 Kohlenstoffatomen, Cycloalkyl mit bis zu 6 Kohlenstoffatomen (z. B. Cyclopropyl, Cyclopentyl oder Cyclohexyl) oder Phenyl stehen, wobei letzteres gegebenenfalls durch Halogen, Alkyl oder Halogenoalkyl mit 1 bis 5 Kohlenstoffatomen, Alkoxy oder Halogenalkoxy mit 1 bis 4 Kohlenstoffatomen, Nitro, Phenyl oder Phenoxy substituiert ist; sowie die Säureadditionssalze und Metallkomplexe davon.

2. Triazolverbindung nach Anspruch 1, worin $R^1$ und $R^2$ für Alkyl mit 1 bis 4 Kohlenstoffatomen, Phenyl oder Halogenophenyl stehen.

3. Triazolverbindung nach Anspruch 1 oder 2, worin $R^1$ und $R^2$ beide für Phenyl, 2-, 3- oder 4-Chlorophenyl, 2,4- oder 2,6-Dichlorophenyl, 2-, 3- oder 4-Fluorophenyl, 2,6-Difluorophenyl, 2-, 3- oder 4-Bromophenyl, 2-Chloro-4-fluorophenyl, 2-Chloro-6-fluorophenyl, 2-, 3- oder 4-Methoxyphenyl, 2,4-Dimethoxyphenyl, 2-, 3- oder 4-Ethoxyphenyl, 2-, 3- oder 4-Nitrophenyl, 2-, 3- oder 4-Trifluoromethylphenyl, 2-, 3- oder 4-Phenoxyphenyl oder 2-, 3- oder 4-Phenylphenyl (2, 3- oder 4-Biphenyl) stehen.

4. Verfahren zur Herstellung einer Verbindung, wie sie in einem der Ansprüche 1 bis 3 definiert ist, bei welchem eine Verbindung der allgemeinen Formel (II)

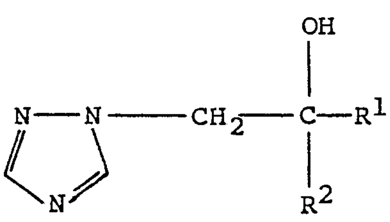

formel (II)

worin $R^1$ und $R^2$ die in einem der Ansprüche 1 bis 3 angegebenen Bedeutungen besitzen, unter sauren Bedingungen durch Rückfluß in einem Lösungsmittel dehydratisiert wird.

5. Verfahren nach Anspruch 4, bei welchem die Verbindung der allgemeinen Formel (II) dadurch hergestellt werden, daß man eine Verbindung der allgemeinen Formeln (III) oder (IV)

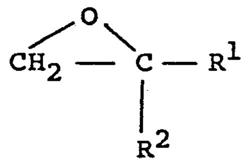

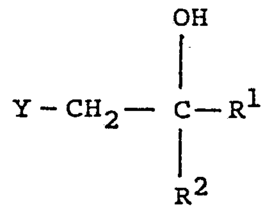

formel (III) formel (IV)

worin $R^1$ und $R^2$ die in einem der Ansprüche 1 bis 3 angegebenen Bedeutungen besitzen und Y für ein Halogenatom steht, mit 1,2,4-Triazol entweder in Gegenwart eines säurebindenden Mittels oder in Form eines seiner Alkalimetallsalze in einem Lösungsmittel umsetzt.

6. Verfahren nach Anspruch 5, bei welchem die Verbindung der allgemeinen Formel (III) oder (IV) bei 20 bis 100°C mit dem Natriumsalz von 1,2,4-Triazol (das Salz kann durch Zugabe von entweder Natriumhydrid oder Natriummethoxid zu 1,2,4-Triazol hergestellt werden) in einem Lösungsmittel, wie z. B. Acetonitril, Methanol, Ethanol oder Dimethylformamid, umgesetzt und das Produkt durch Einschütten des Reaktionsgemischs in Wasser und Umkristallisieren isoliert wird.

7. Verfahren nach Anspruch 5, bei welchem die Verbindungen der allgemeinen Formel (III) und (IV) dadurch hergestellt werden, daß man eine Verbindung der allgemeinen Formel (Va) oder (Vb)

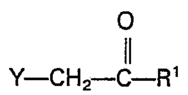

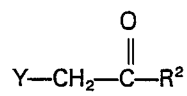

Formel (Va) Formel (Vb)

worin $R^1$, $R^2$ und Y die angegebenen Bedeutungen besitzen, mit einer Grignard-Verbindung der allgemeinen Formel (VIa) bzw. (VIb)

$Z—Mg—R^1$ $Z—Mg—R^2$

Formel (VIa) Formel (VIb)

worin $R^1$ und $R^2$ die oben angegebenen Bedeutungen besitzen und Z für ein Halogen (vorzugsweise Chlor, Brom oder Jod) steht, in einem Lösungsmittel, wir z.B. Diethylether oder Tetrahydrofuran, umsetzt.

8. Verfahren nach Anspruch 5, bei welchem die Verbindungen der allgemeinen Formel (III), worin $R^1$ und $R^2$, welche gleich oder verschieden sein können, jeweils für substituiertes Phenyl stehen, dadurch hergestellt werden, daß man die entsprechende Benzophenonverbindung der allgemeinen Formel (VII)

$R^1—CO—R^2$ Formel (VII)

worin $R^1$ und $R^2$ die oben angegebenen Bedeutungen besitzen, mit Dimethyl-oxosulfonium-methylid oder Dimethylsulfonium-methylid umsetzt.

9. Fungicide Zusammensetzung, welche als aktiven Bestandteil eine Verbindung nach einem der Ansprüche 1 bis 3 oder ein Salz oder einen Komplex davon und ein Träger- oder Verdünnungsmittel hierfür enthält.

10. Verfahren zur Bekämpfung von Pilzerkrankungen an einer Pflanze, bei welchem auf die Pflanze, den Samen der Pflanze oder die Umgebung der Pflanze oder des Samens eine Verbindung oder ein Salz oder ein Komplex davon, wie sie in einem der Ansprüche 1 bis 3 definiert sind, oder eine Zusammensetzung nach Anspruch 9 aufgebracht wird.

11. Pharmazeutische oder veterinäre fungicide Zusammensetzung, welche eine Verbindung oder ein Salz, einen Metallkomplex, einen Ether oder einen Ester davon, wie sie in einem der Ansprüche 1 bis 3 definiert sind, gemeinsam mit einem pharmazeutisch oder veterinär zulässigen Verdünnungs- oder Trägermittel enthält.

**Revendications**

1. Composé de triazole de formule générale (I):

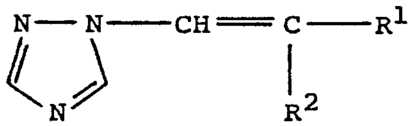

formule (I)

qui est l'isomère cis ou trans, ou un mélange de ces isomères, formule dans laquelle $R^1$ et $R^2$, qui peuvent être égaux ou différents, sont des groupes alkyle ayant 1 à 6 atomes de carbone, cycloalkyle ayant jusqu'à 6 atomes de carbone (par exemple cyclopropyle, cyclopentyle ou cyclohexyle) ou le groupe phényle éventuellement substitué avec un halogène, un radical alkyle ou un radical halogénalkyle ayant 1 à 5 atomes de carbone, alkoxy ou halogénalkoxy ayant 1 à 4 atomes de carbone, nitro, phényle ou phénoxy; et ses sels d'addition d'acides et ses complexes métalliques.

2. Composé de triazole suivant la revendication 1, dans lequel $R^1$ et $R^2$ sont des groupes alkyle ayant 1 à 4 atomes de carbone, phényle ou halogénophényle.

3. Composé de triazole suivant la revendication 1 ou 2, dans lequel $R^1$ et $R^2$ sont tous deux un groupe phényle, 2-, 3- ou 4-chlorophényle, 2,4- ou 2,6-dichlorophényle, 2-, 3- ou 4-fluorophényle, 2,6-difluorophényle, 2-, 3- ou 4-bromophényle, 2-chloro-4-fluorophényle, 2-chloro-6-fluorophényle, 2-, 3- ou 4-méthoxyphényle, 2,4-diméthoxyphényle, 2-, 3- ou 4-éthoxyphényle, 2-, 3- ou 4-nitrophényle, 2-, 3- ou 4-trifluorométhylphényle, 2-, 3- ou 4-phénoxyphényle ou 2-, 3- ou 4-phénylephényle (2-, 3- ou 4-biphényle).

4. Procédé de préparation d'un composé tel que défini dans l'une quelconque des revendications 1 à 3, qui consiste à déshydrater un composé de formule générale (II):

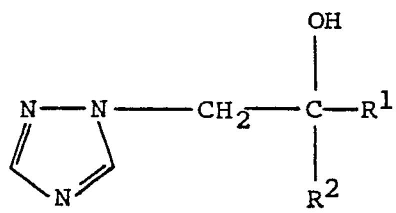

formule (II)

dans laquelle $R^1$ et $R^2$ sont tels que définis dans l'une quelconque des revendications 1 à 3 dans des conditions acides, par chauffage au reflux dans un solvant.

5. Procédé suivant la revendications 5, dans lequel des composés de formule générale (II) sont produits par réaction d'un composé de formule générale (III) ou (IV):

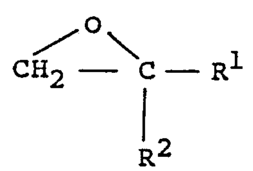

formule (III)

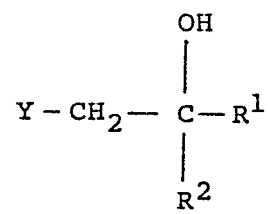

formule (IV)

dans laquelle $R^1$ et $R^2$ sont tels que définis dans l'une quelconque des revendications 1 à 3 et Y est un atome d'halogène, avec le 1,2,4-triazole en présence d'un accepteur d'acide ou sous la forme de l'un de ses sels de métaux alcalins dans un solvant.

6. Procédé suivant la revendication 5, dans lequel le composé de formule générale (III) ou (IV) est amené à réagir à 20—100°C avec le sel de sodium du 1,2,4-triazole (le sel peut être préparé par addition d'hydrure de sodium ou de méthylate de sodium à du 1,2,4-triazole) dans un solvant tel que l'acétonitrile, le méthanol, l'éthanol ou le diméthylformamide, et le produit est isolé en versant le mélange réactionnel dans de l'eau et en le faisant recristalliser.

7. Procédé suivant la revendication 5, dans lequel les composés de formules générales (III) et (IV) sont préparés par réaction d'un composé de formule générale (Va) ou (Vb):

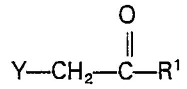

formule (Va)

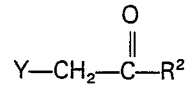

formule (Vb)

où $R^1$, $R^2$ et Y ont les définitions données, avec, respectivement, un composé de Grignard de formule générale (VIa) ou (VIb):

Z—Mg—$R^1$ formule (VIa)

Z—Mg—$R^2$ formule (VIb)

dans laquelle $R^1$ et $R^2$ sont tels que définis ci-dessus et Z est un halogène (de préférence le chlore, le brome ou l'iode) dans un solvant tel que l'éther de diéthyle ou le tétrahydrofuranne.

8. Procédé suivant la revendication 5, dans lequel les composés de formule générale (III) dont chacun des groupes $R^1$ et $R^2$, qui peuvent être égaux ou différents, représente un groupe phényle substitué, sont préparés par réaction de la benzophénone appropriée de formule générale (VII)

$R^1$—CO—$R^2$ formule (VII)

dans laquelle $R^1$ et $R^2$ sont tels que définis ci-dessus avec le méthylure de diméthyloxysulfonium ou le méthylure de diméthylsulfonium.

9. Composition fongicide, comprenant comme ingrédient actif un composé suivant l'une quelconque des revendications 1 à 3 ou un sel ou complexe de ce composé; et un support ou diluant approprié.

10. Procédé pour combattre des maladies fongiques chez une plante, procédé qui consiste à appliquer à la plante, à ses graines ou au lieu où se trouve la plante ou la graine un composé ou un sel ou complexe de ce composé tel que défini dans l'une quelconque des revendications 1 à 3 ou une composition suivant la revendication 9.

11. Composition fongicide pharmaceutique ou vétérinaire, qui comprend un composé ou un sel, un complexe métallique, un éther ou un ester de ce composé tel que défini dans l'une quelconque des revendications 1 à 3 en association avec un diluant ou support acceptable du point de vue pharmaceutique ou vétérinaire.